# FASCICULE DE BREVET EUROPEEN

(11) **EP 2 609 882 B1**
(45) Date de publication et mention de la délivrance du brevet: **21.11.2018**
(21) Numéro de dépôt: 12197934.8
(22) Date de dépôt: 19.12.2012
(51) Int. Cl.: A61B 17/70

(54) **Dispositif de fixation vertébrale**
Wirbelbefestigungsvorrichtung
Vertebral fixing device

(30) Priorité: 29.12.2011 FR 1162554
(43) Date de publication de la demande: 03.07.2013
(73) Titulaire: Cousin Biotech, 59117 Wervicq Sud (FR)
(72) Inventeur: Deneuvillers, Guy, 62155 MERLIMONT (FR)
(74) Mandataire: Balesta, Pierre

(56) Documents cités:
- EP-A1- 0 597 258
- EP-A1- 2 047 813
- EP-A1- 2 138 122
- FR-A1- 2 704 745

## Description

La présente invention concerne un dispositif de fixation vertébrale implantable, notamment pour l'ostéosynthèse vertébrale, par exemple en cas de fracture(s) du rachis, ainsi que pour le traitement des scolioses et de façon générale pour la correction des courbures anormales du rachis.

Le rachis ou colonne vertébrale est divisé en trois étages. L'étage cervicale ou rachis cervicale est composé de sept vertèbres (C1-C7), l'étage dorsale ou rachis dorsal est composé de douze vertèbres (D1-D12). L'étage lombaire ou rachis lombaire est composé de cinq vertèbres (L1-L5). Le rachis dorsal a une courbure particulière (cyphose) opposée à celle des rachis cervical et lombaire (lordose). Chaque vertèbre du rachis est composée d'un corps vertébral en avant, lequel est relié à un fragment osseux, allongé en arrière (apophyse épineuse), par des arcs osseux postérieurs (un de chaque côté) et des lames osseuses (une de chaque côté). Entre les arcs postérieurs et les lames se trouvent les apophyses transverses (une de chaque côté) sur lesquelles s'insèrent différents muscles. Les vertèbres sont reliées entre-elles par un système amortisseur fibro-cartilagineux de forme discoïde appelé disque inter-vertébral. L'empilement des vertèbres délimite un canal en arrière des corps vertébraux nommé canal médullaire dans lequel s'étend la moelle épinière (jusqu'à la deuxième vertèbre lombaire L2).

On connait pour le traitement des scolioses, en particulier des scolioses sévères, une technique opératoire consistant à rapporter sur la zone du rachis dont on souhaite corriger la courbure, deux tiges de liaison cintrées disposées de part et d'autre de chaque vertèbre de ladite zone, et solidarisées au corps vertébral par des fils de liaison, notamment métalliques. Chaque fil passe sur au moins une portion du corps vertébral, notamment sous une lame osseuse, puis enserre une tige de liaison, le chirurgien solidarise alors les extrémités libres du fil de liaison en les enroulant sur elles-mêmes provoquant alors le rapprochement de la portion du corps vertébral vers la tige de liaison et la solidarisation de la tige de liaison à cette portion du corps vertébral. Il procède ainsi pour chaque vertèbre de la zone du rachis à corriger.

Cette technique présente l'inconvénient que le serrage est irréversible. Les vertèbres étant mobiles les unes par rapport aux autres, le fil métallique bouge de la position dans laquelle il a été implanté et cisaille les zones sur lesquelles il est en appui, ce qui est dangereux s'agissant de zones d'implantation au plus près de la moelle épinière. De plus, les extrémités libres du fil de liaison coupées sont saillantes.

On connaît également des dispositifs de fixation vertébrale, notamment pour l'ostéosynthèse vertébrale, comprenant généralement des organes d'ancrages osseux, tels que des vis pédiculaires, des pinces ou des crochets ainsi qu'une ou deux tiges de liaison destinées à être reliées à ces organes d'ancrage, et des pièces de connexion de cette ou de ces tiges de liaison à ces organes d'ancrage. Le matériel peut comprendre également des traverses réglables, qui relient transversalement deux tiges de liaison parallèles pour maintenir ces tiges de liaison l'une par rapport à l'autre.

On connaît également des dispositifs de fixation vertébrale dans lesquels la solidarisation de la tige de liaison n'est pas assurée via des organes d'ancrages osseux mais via un ligament. Le dispositif est alors agencé pour recevoir la tige de liaison et un ligament et en sorte que la tige de liaison et le ligament soient bloqués ensemble ou séparément, le ligament passant autour d'une portion d'un corps vertébral.

Les moyens de blocage de la tige de liaison et du ligament enserrent totalement ces derniers, notamment par pincement, et sont actionnables via une ou plusieurs vis de serrage maintenant serrées deux parties rabattues l'une vers l'autre et délimitant un logement intérieur de forme sensiblement circulaire pour la tige de liaison, éventuellement en contact aussi avec le ligament.

Ces dispositifs sont difficilement réversibles une fois réglés. Il en effet nécessaire d'actionner la ou les vis puis de tirer sur le ligament. De plus, la tige de liaison étant bloquée dans un logement intérieur de forme circulaire, elle ne présente aucune mobilité selon son axe longitudinale. Ce type de dispositif n'autorise aucun mouvement entre la tige de liaison et le ligament.

On connait également EP 2.138.122 A1 ayant pour objet un dispositif de fixation vertébrale implantable décrit comme permettant la stabilisation du sacrum avec au moins la cinquième vertèbre lombaire. Ce dispositif comprend deux éléments longilignes ayant des premières et secondes extrémités ainsi qu'un support de fixation ayant des orifices d'admission et de sortie pour chacun desdits éléments longilignes.

Les premières extrémités des éléments longilignes sont fixés dans l'os du sacrum via des moyens d'ancrage tandis que les secondes extrémités sont bloquées entre les orifices d'admission et de sortie par l'intermédiaire d'une vis ou d'un coin conique par pincement desdites secondes extrémités contre une portion interne du support de fixation.

On retrouve encore un moyen de blocage des éléments longilignes difficilement réversible une fois réglé.

Enfin, ces dispositifs de fixation sont complexes à fabriquer et couteux, ce qui peut dans certains cas influencer le choix de la technique opératoire.

La présente invention a pour objet un dispositif de fixation vertébrale palliant les problèmes précités, facile à mettre oeuvre et peu onéreux à fabriquer comprenant un élément longiligne sensiblement plat de largeur principale déterminée (l), ayant des première et seconde extrémités, destiné à être disposé autour d'une portion d'un corps vertébral. Avantageusement, le dispositif comprend un support de fixation ayant des parties avant et arrière et une zone d'attache à laquelle ladite première extrémité est solidarisée ou apte à être solidarisée ainsi qu'un premier élément de blocage, monté sur ledit support de fixation en sorte d'être apte à translater entre lesdites parties avant et arrière, et délimitant des zones de passage avant et arrière avec lesdites parties avant et arrière, respectivement, permettant le passage de ladite seconde extrémité libre. Le dispositif comprend également un moyen d'appui et de guidage dudit élément longiligne. En fonctionnement la seconde extrémité libre passe au moins une fois dans lesdites zones avant et arrière et sur ledit moyen d'appui et de guidage en passant autour, au moins partiellement, d'une tige de liaison et d'une portion d'un corps vertébral pour la solidarisation amovible de ladite tige de liaison avec ledit support de fixation. La traction exercée sur la seconde extrémité libre permet le déplacement du premier élément de blocage vers la partie avant et corrélativement le rapprochement de ladite portion du corps vertébral vers ladite tige de liaison.

Avantageusement, la tige de liaison et l'élément longiligne ne sont pas bloqués individuellement ou ensemble par des vis ou d'autres moyens équivalents, ce qui laisse la possibilité à la tige de liaison d'accepter des micromouvements selon son axe sensiblement longitudinal et confère une certaine mobilité de la tige de liaison par rapport à l'élément longiligne. Cette mobilité est bien sûr réduite, il s'agit de mouvements de très faible amplitude mais tout de même présents pour améliorer le confort du patient.

En outre, contrairement au dispositif décrit dans EP 2.138.122 A1, la traction exercée sur la seconde extrémité libre de l'élément longiligne permet le déplacement du premier élément de blocage vers la partie avant et corrélativement le coulissement de l'élément longiligne dans lesdites zones de passage avant et arrière.

Le desserrage de l'élément longiligne peut facilement être opéré en écartant, manuellement ou à l'aide d'un outil, l'élément de blocage de la partie avant du support de fixation.

Dans EP 2.138.122 A1, la traction sur l'une des extrémités libres d'un des deux éléments longilignes n'entraine le déplacement en translation d'aucun élément du support de fixation. Il est nécessaire de dévisser la vis pour libérer et régler la tension exercée par l'élément longiligne.

En fonctionnement, la tige de liaison est tout d'abord solidarisée au support de fixation par l'élément longiligne, lequel passe autour, au moins partiellement, de la tige de liaison et d'une portion du corps cylindrique en passant sur le moyen d'appui et de guidage et dans les zones de passage avant et arrière pour la solidarisation amovible de la tige de liaison et de ladite portion du corps vertébrale audit support. Lorsqu'on exerce une traction sur la seconde extrémité libre de l'élément longiligne, la tige de liaison se rapproche du moyen d'appui et de guidage, puis l'élément longiligne déplace le premier élément de blocage vers la partie avant, tout en glissant autour du premier élément de blocage et sur le moyen d'appui et de guidage provoquant alors le rapprochement de la tige de liaison vers la portion du corps vertébrale. Lorsqu'on cesse d'exercer une traction sur la seconde extrémité libre de l'élément longiligne, le premier élément de blocage reste bloqué par rapport au moyen d'appui et de guidage.

Le moyen d'appui et de guidage a ainsi pour fonction d'exercer une contre-tension lorsqu'une traction est exercée sur la seconde extrémité libre, sans que le chirurgien n'ait lui-même à l'exercer manuellement, permettant le coulissement de l'élément longiligne sur le moyen d'appui et de guidage et dans les zones de passage avant et arrière, et dès lors le rapprochement de la tige de liaison de la portion du corps vertébral.

De préférence, le moyen d'appui et de guidage a une forme arrondie facilitant le passage en appui sur ce dernier de l'élément longiligne.

De préférence, l'élément longiligne s'étend dudit moyen d'appui et de guidage en passant autour, au moins partiellement de la tige de liaison, la forme arrondie dudit moyen facilite ainsi la solidarisation de la tige de liaison par ledit élément longiligne.

Il est nécessaire que l'élément longiligne soit sensiblement plat en sorte que les frottements générés par le passage dudit élément sur les parties avant, arrière, le premier élément de blocage et le moyen d'appui et de guidage participent au blocage de l'élément longiligne dans les zones de passage avant et arrière.

On comprend par une portion du corps vertébral, notamment toute portion d'un arc osseux postérieur, d'une lame osseuse ou d'une apophyse transverse.

Dans une variante, le support de fixation comprend une partie de forme générale semi-cylindrique ouverte formant un logement apte à recevoir une tige de liaison.

L'élément longiligne passe ainsi autour de la portion de la tige de liaison qui n'est pas recouverte par le support de fixation.

De préférence, le support de fixation présente une forme générale semi-cylindrique ouverte formant un logement apte à recevoir une tige de liaison.

Dans une variante, ladite partie de forme générale semi-cylindrique ouverte comprend une portion de sa surface externe faisant office dudit moyen de guidage et d'appui pour ledit élément longiligne.

De préférence, la seconde extrémité libre passe ainsi au moins partiellement autour de la tige de liaison et autour d'une portion du corps vertébral, sur une portion de la surface externe du support de fixation, puis dans la zone de passage arrière et enfin dans la zone de passage avant.

La tige de liaison est donc solidarisée au support de fixation en étant plaquée dans le logement du support de fixation soit par l'élément longiligne directement, soit par la portion du corps vertébrale via la traction exercée sur la seconde extrémité de ledit élément longiligne.

L'élément longiligne vient ainsi en appui sur ladite portion de la surface externe du support de fixation, et est guidée de par sa forme arrondie vers la zone de passage arrière.

Dans une variante, la partie arrière fait office de ladite zone d'attache.

Dans une variante, le dispositif comprend un second élément de blocage monté en sorte d'être apte à translater entre les parties avant et arrière délimitant avec le premier élément de blocage une zone de passage intermédiaire, ledit second élément de blocage faisant office dudit moyen d'appui et de guidage pour l'élément longiligne.

Les zones de passage avant, intermédiaire et arrière sont délimitées entre la partie avant et le premier élément de blocage, les premier et second éléments de blocage, et le second élément de blocage et la partie arrière respectivement.

De préférence, la seconde extrémité libre de l'élément longiligne passe dans cet ordre autour de la tige de liaison puis dans la zone de passage intermédiaire, la zone de passage arrière, autour d'une portion du corps vertébral, puis dans la zone de passage intermédiaire et enfin dans la zone de passage avant.

Dans une sous-variante, le second élément de blocage fait office de ladite zone d'attache.

Dans une variante, le support de fixation comprend deux parties latérales entre lesquelles ledit premier élément de blocage, éventuellement ledit second élément de blocage, est monté coulissant.

Dans une variante, la distance interne (d) séparant les parties latérales est de l'ordre de celle de la largeur principale (l) dudit élément longiligne ou inférieure.

Cette disposition permet d'augmenter les zones de frottements entre l'élément longiligne et les parties avant, arrière et latérales, le premier élément de blocage, éventuellement le second élément de blocage, afin d'améliorer le blocage de l'élément longiligne dans les zones de passage avant et arrière, éventuellement intermédiaire.

Dans une variante, la partie avant du support de fixation est dimensionnée et agencée par rapport au premier élément de blocage en sorte de faire office de butée avant pour ledit premier élément de blocage, de préférence les bords gauche et droit de la partie avant se projetant de part et d'autre des parties latérales font office de butée aux bords gauche et droit du premier élément de blocage.

Dans une variante, le premier élément de blocage, éventuellement le second élément de blocage, comprend une ouverture centrale dont les bords latéraux internes sont ménagés en sorte de recevoir les parties latérales du support de fixation et faire office de guide pour la coulisse du premier élément de blocage, éventuellement du second élément de blocage, sur les parties latérales.

Dans une variante, la périphérie supérieure de l'ouverture centrale débouchant vers la partie avant est délimitée par des bords gauche et droit, et le bord gauche se projette de ladite périphérie supérieure en sorte que seul le bord gauche du premier élément de blocage vienne en butée contre la partie avant du support de fixation ménageant un jeu entre le bord droit et la partie avant.

Le jeu ménagé entre le bord droit et la partie avant du support de fixation permet de diminuer la force du pincement et donc de limiter le cisaillement de l'élément longiligne pincé entre le premier élément de blocage et la partie avant du support de fixation lorsque des tensions opposées sont exercées sur les parois internes de la boucle.

La résistance à rupture de l'élément longiligne est ainsi améliorée.

Cette disposition peut éventuellement s'appliquer au second élément de blocage, lequel vient en butée contre le premier élément de blocage ménageant alors un jeu entre son bord droit et le second élément de blocage.

Dans une variante, le premier élément de blocage, éventuellement le second élément de blocage, comprend des rainures verticales, en particulier orientées de la partie arrière vers la partie avant et débouchant sur la périphérie supérieure, et éventuellement la périphérie inférieure, de l'ouverture centrale en sorte de ménager des dents de pincement sur la périphérie supérieure, et éventuellement la périphérie inférieure.

Le blocage de l'élément longiligne dans les zones de passage avant et arrière, et éventuellement intermédiaire, est ainsi amélioré.

Dans une variante, l'ouverture centrale du premier élément de blocage, éventuellement du second élément de blocage, débouchant vers la partie avant du support comporte selon au moins l'un de ses bords gauche ou droit des dents en forme de dents de scie.

Le blocage de l'élément longiligne dans les zones de passage avant et arrière, et éventuellement intermédiaire, est ainsi amélioré.

Dans une variante, le support de fixation est dans un matériau sélectionné seul ou en combinaison parmi les matériaux suivants : acier inoxydable, titane, céramique, polypropylène, polyéthylène, polyéthylène haute densité, le polyétheréthercétone (PEEK), polyéthercétonecétone (PEKK).

Le support de fixation peut être moulé ou usiné dans lesdits matériaux.

Dans une variante, l'élément longiligne est obtenu par la mise en forme de fils multi-filamentaires et/ou de monofilaments par tissage, tricotage ou tressage, de préférence par tressage de fils multi-filamentaires.

Dans une variante, l'élément longiligne est une tresse tubulaire dont les première et seconde extrémités ont été cousues pour la mise en forme à plat de la tresse tubulaire.

Dans une variante, la seconde extrémité est confectionnée en forme de pointe, de préférence comporte une boucle de préhension.

Dans une variante, l'élément longiligne comprend une zone centrale disposée entre des zones latérales et en ce que les zones latérales présentent un coefficient de friction moins élevé que la zone centrale.

La présente invention sera mieux comprise à la lecture de trois exemples de réalisation, cités à titre non limitatif, et illustrés par les figures suivantes, annexées à la présente et dans lesquelles :
- la figure 1A est une représentation schématique d'un premier exemple de support de fixation selon l'invention;
- la figure 1B est une représentation schématique d'une première variante du premier exemple de support de fixation représenté à la figure 1A ;
- la figure 1C est une représentation schématique d'une seconde variante du premier exemple de support de fixation représenté à la figure 1A ;
- la figure 2 est une représentation schématique d'un premier exemple de dispositif de fixation vertébrale comprenant le support de fixation représenté à la figure 1C en fonctionnement ;
- la figure 3 est représentation schématique d'un second exemple de support de fixation selon l'invention ;
- la figure 4 est une représentation schématique d'un second exemple de dispositif de fixation vertébrale selon l'invention comprenant le support de fixation représenté à la figure 3, en fonctionnement ;
- la figure 5 est une représentation schématique d'un troisième exemple de support de fixation selon l'invention ;

Les figures 6A et 6B représentent de façon schématique un troisième exemple de dispositif de fixation vertébrale selon l'invention comprenant le support de fixation représenté à la figure 5.

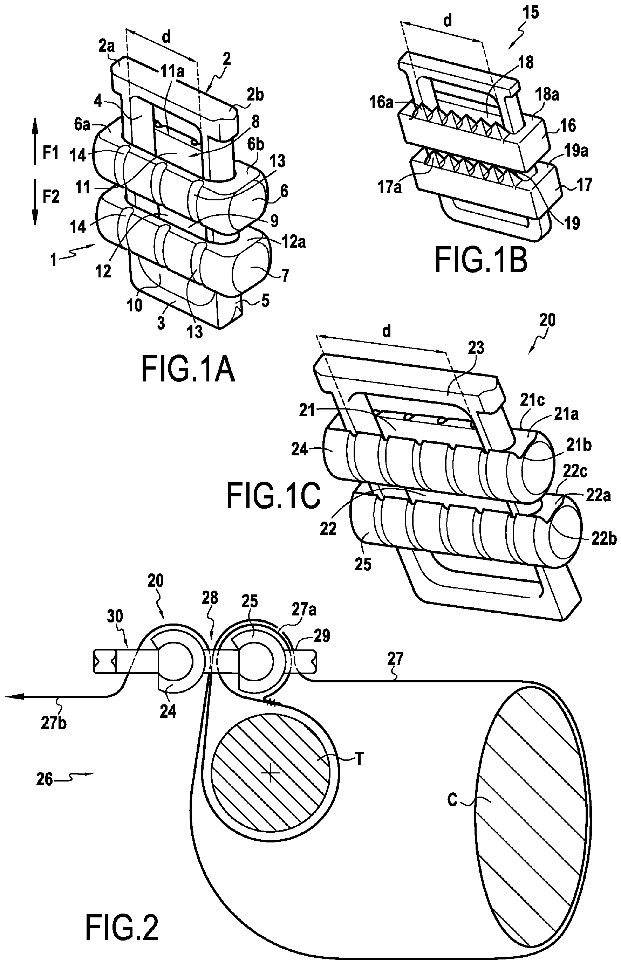

La figure 1A représente un premier support de fixation 1 ayant des parties avant 2 et arrière 3 et deux parties latérales 4,5. Le support de fixation 1 comprend un premier élément de blocage 6 et un second élément de blocage 7 montés sur ledit support de fixation 1 en sorte d'être apte à translater entre lesdites parties avant 2 et arrière 3 délimitant des zones de passage avant 8, intermédiaire 9 et arrière 10 permettant le passage de la seconde extrémité libre d'un élément longiligne. Dans cet exemple précis, les premier 6 et second 7 éléments de blocage sont montés coulissant entre les parties latérales 4,5. Le second élément de blocage 7 fait office de moyen d'appui et de guidage d'un élément longiligne. Le second élément de blocage 7 fait également office de zone d'attache à laquelle la première extrémité d'un élément longiligne peut être solidarisée.

De préférence, la distance interne (d) séparant les parties latérales 4,5 est de l'ordre de celle de la largeur principale (l) dudit élément longiligne ou inférieur.

La partie avant 2 du support de fixation 1 est dimensionnée et agencée par rapport au premier élément de blocage 6 en sorte de faire office de butée avant pour ledit premier élément de blocage 6, de préférence les bords gauche 2a et droit 2b de la partie avant 2 se projetant de part et d'autre des parties latérales 4,5 font office de butée aux bords gauche 6a et droit 6b du premier élément de blocage 6.

Les premier 6 et second 7 éléments de blocage comprennent une ouverture centrale 11,12 dont les bords latéraux internes sont ménagés en sorte de recevoir les parties latérales 4,5 du support de fixation 1 et faire office de guide pour la coulisse des premier 6 et second 7 éléments de blocage sur les parties latérales 4,5.

Le premier élément de blocage 6, et le second élément de blocage 7, comprennent des rainures verticales 13, orientées de la partie arrière 3 vers la partie avant 2, et débouchant sur les périphéries supérieures 11a, 12a, et éventuellement sur les périphéries inférieures, des ouvertures centrales 11,12 en sorte de ménager des dents de pincement 14 sur leurs périphéries supérieures 11a, 12a, et éventuellement leurs périphéries inférieures.

Le support de fixation 15 représenté à la figure 1B diffère du support de fixation 1 représenté à la figure 1A en ce que les premier 16 et second 17 éléments de blocage ne comportent pas de rainures verticales mais au moins selon l'un de leurs bords gauche 16a,17a, des dents en forme de dents de scie s'étendant des périphéries supérieures 18a,19a des ouvertures centrales 18,19.

Le support de fixation 20 représenté à la figure 1C diffère du support de fixation 1 représenté à la figure 1A en ce que les périphéries supérieures 21a,22a des ouvertures centrales 21,22 débouchant vers la partie avant 23 sont délimitées par des bords gauches 21b,22b et droits 21c,22c. Les bords gauches 21b,22b se projettent desdites périphéries supérieures 21a,22a en sorte que seuls les bords gauches 21b,22b des premier 24 et second 25 éléments de blocage viennent en butée contre la partie avant 23 et le premier élément de blocage 24, respectivement, du support de fixation 20 ménageant un jeu entre les bords droits 21c,22c et la partie avant 23 et le premier élément de blocage 24, respectivement.

La figure 2 représente de façon schématique, en fonctionnement, un dispositif de fixation vertébrale 26 comprenant un exemple de support de fixation selon l'invention, de préférence le support de fixation 20 représenté à la figure 1C.

Le dispositif de fixation vertébrale 26 comprend également un élément longiligne 27 sensiblement plat de largeur principale déterminée (l), ayant des première 27a et seconde 27b extrémités, dont la première extrémité 27a est solidarisée au second élément de blocage 25 faisant office de zone d'attache.

En fonctionnement, le chirurgien passe la seconde extrémité libre 27b de l'élément longiligne 27 autour de la tige de liaison (T) puis dans la zone de passage intermédiaire 28 et dans la zone de passage arrière 29, il exerce alors une traction sur la seconde extrémité libre 27b en sorte de rapprocher la tige de liaison (T) du support de fixation 20 et solidariser ladite tige de liaison au support 20. La seconde extrémité libre 27b est ensuite passée autour d'une portion d'un corps vertébral (C), puis de nouveau dans la zone de passage intermédiaire 28 en passant partiellement autour de la tige de liaison (T) et enfin dans la zone de passage avant 30. La traction exercée sur la seconde extrémité libre 27b permet le déplacement des premier 24 et second 25 éléments de blocage vers la partie avant 23, et corrélativement le rapprochement de la portion du corps vertébral (C) vers la tige de liaison (T).

Le second élément de blocage 25, ayant coulissé avec le premier élément de blocage 24 vers la partie avant 23, bloque le premier élément de blocage 24 dans la position dans laquelle il est plaqué contre la partie avant 23. L'élément longiligne 27 et la tige de liaison (T) sont ainsi parfaitement solidarisés au support de fixation 20.

Lorsqu'il est nécessaire de réajuster le serrage, il suffit de tirer sur la seconde extrémité libre 27b - lorsqu'on souhaite rapprocher encore la portion du corps vertébral (C) de la tige de liaison (T) - et/ou de desserrer l'élément longiligne 27 en séparant manuellement le premier élément de blocage 24 du second élément de blocage 25.

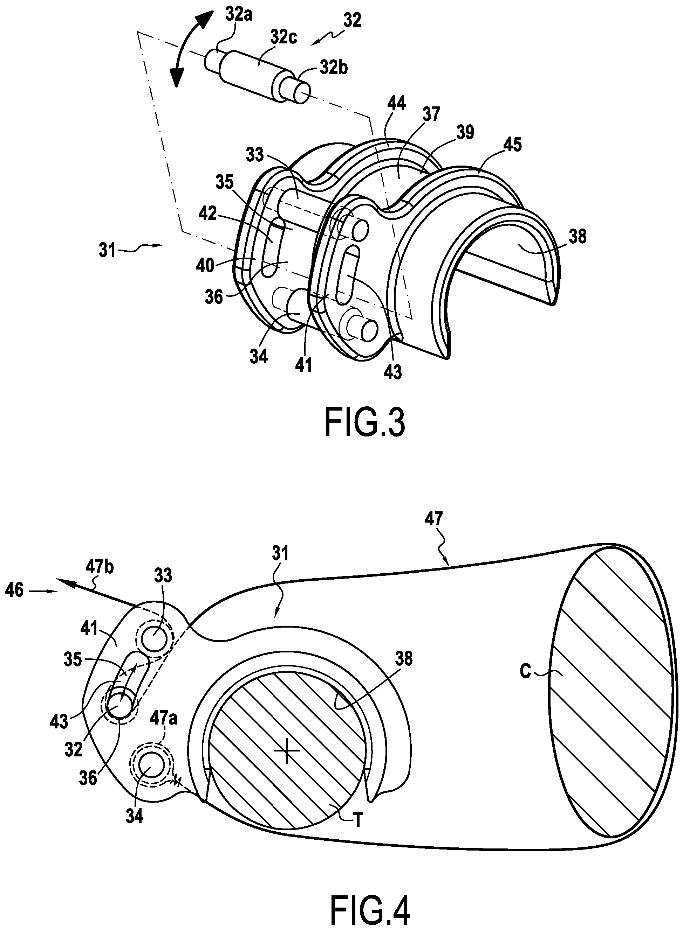

La figure 3 représente un second exemple de support de fixation 31 selon l'invention comprenant un premier élément de blocage 32 monté sur ledit support de fixation 31 en sorte d'être apte à translater entre les parties avant 33 et arrière 34 délimitant des zones de passage avant 35 et arrière 36 avec ledit premier élément de blocage 32 permettant le passage de la seconde extrémité libre d'un élément longiligne. Le support de fixation 31 comporte une partie 37 ayant une forme générale semi-cylindrique ouverte formant un logement 38 apte à recevoir une tige de liaison dont une portion 39 de la surface externe fait office de moyen de guidage et d'appui pour ledit élément longiligne. La partie arrière 34 fait office de zone d'attache sur laquelle la première extrémité d'un élément longiligne peut être solidarisée. Le support de fixation 31 comprend également deux parties latérales 40,41 entre lesquelles ledit premier élément de blocage 32 est monté coulissant. Dans cet exemple précis, les parties latérales 40,41 comprennent chacune une rainure traversante 42,43 dans lesquelles sont disposées les extrémités 32a,32b du premier élément de blocage 32. L'élément de blocage 32 présente une partie centrale 32c ayant un diamètre supérieur à celui des extrémités 32a,32b en sorte qu'une fois emboîté en force dans les rainures 42,43 des parties latérales 40,41, il puisse coulisser dans ces dernières tout en étant retenu entre-elles.

Les parties latérales 40,41 sont dans le prolongement de deux bordures 44,45 se projetant de la surface externe du support de fixation 31 et permettant d'empêcher que l'élément longiligne passant sur le moyen d'appui et de guidage 39 ne glissent sur les côtés du support de fixation 31.

Le dispositif de fixation 46 représenté à la figure 4 en fonctionnement comprend le support de fixation 31 décrit à la figure 3. Ce dispositif 46 comprend également un élément longiligne 47 dont la première extrémité 47a est solidarisée à la partie arrière 34 du support de fixation 31. La seconde extrémité libre 47b passe partiellement autour de la tige de liaison (T) puis autour d'une portion d'un corps vertébral (C), dans la zone de passage arrière 36, et enfin la zone de passage avant 35. Lorsqu'une traction est exercée ensuite sur la seconde extrémité libre 47b, elle entraîne le plaquage et donc la solidarisation de la tige de liaison (T) dans le logement 38 du support de fixation 31, le déplacement du premier élément de blocage 32 vers la partie avant 33, et corrélativement le rapprochement de la portion du corps vertébral (C) de la tige de liaison (T). Le moyen d'appui et de guidage formé par une portion 39 de la surface externe du support de fixation 31 permet de bloquer le premier élément de blocage 32 contre la partie avant 33 et ainsi de solidariser la tige de liaison (T) et la portion du corps vertébral (C) au support de fixation 31.

Le réglage du serrage de l'élément longiligne 47 s'effectue soit en tirant de nouveau sur la seconde extrémité libre 47b pour de nouveau rapprocher la tige de liaison (T) du corps vertébral (C) et/ou soit en écartant le premier élément de blocage 32 de la partie avant 33.

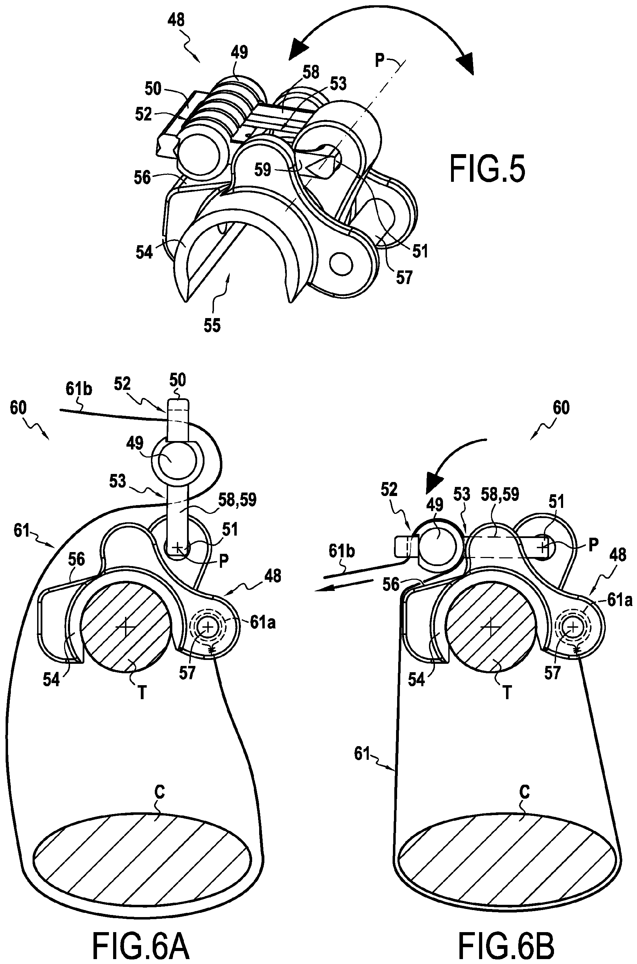

La figure 5 représente un troisième exemple de support de fixation 48 selon l'invention comprenant un premier élément de blocage 49 monté sur ledit support de fixation 48 en sorte d'être apte à translater entre des parties avant 50 et arrière 51 délimitant des zones de passage avant 52 et arrière 53 avec ledit premier élément de blocage 49 permettant le passage de la seconde extrémité libre d'un élément longiligne. Le support de fixation 48 comporte une partie 54 ayant une forme générale semi-cylindrique ouverte formant un logement 55 apte à recevoir une tige de liaison (T) dont une portion 56 de la surface externe fait office de moyen de guidage et d'appui pour ledit élément longiligne. Le support de fixation comprend une zone d'attache 57 à laquelle peut être solidarisée une première extrémité d'un élément longiligne. Le support de fixation 48 comprend également deux parties latérales 58,59 entre lesquelles ledit premier élément de blocage 49 est monté coulissant. La partie arrière 51 est montée en rotation selon l'axe (P). Le premier élément de blocage 49 peut ainsi être disposé selon un axe sensiblement perpendiculaire à la portion 56 de la surface externe du support 48 faisant office de moyen de guidage et d'appui ce qui facilite le passage d'un élément longiligne sur ledit moyen d'appui et de guidage et dans les zones de passage avant 52 et arrière 53.

Les figures 6A et 6B représentent un dispositif de fixation vertébrale 60 comprenant le support de fixation 48 représenté à la figure 5 et un élément longiligne 61 dont la première extrémité 61a est solidarisée à la zone d'attache 57. En fonctionnement, la seconde extrémité 61b de l'élément longiligne 61 passe partiellement autour de la tige de liaison (T) et atour d'une portion du corps vertébral (C) puis dans la zone de passage arrière 53 et enfin dans la zone de passage avant 52. Le réglage du serrage de l'élément longiligne 61 s'effectue de la même façon que pour le support de fixation 31 décrit aux figures 3 et 4.

A la figure 6A, les parties latérales 58,59 sont disposées dans un plan sensiblement perpendiculaire à l'axe (P), ce qui facilite le passage de la seconde extrémité libre 61b. Lorsque le chirurgien exerce une traction sur la seconde extrémité libre 61b, la mise en tension de l'élément longiligne 61 provoque le déplacement du premier élément de blocage vers la partie avant 50 et place les parties latérales 59,58 dans un plan sensiblement parallèle à celui correspondant à l'axe (P) tel que cela est représenté à la figure 6B.

## Revendications

1. Dispositif de fixation vertébrale implantable (26, 46, 60), notamment pour l'ostéosynthèse vertébrale et le traitement des scolioses, comprenant un élément longiligne (27, 47, 61) sensiblement plat de largeur principale déterminée (l), ayant des première (27a, 47a, 61a) et seconde (27b, 47b, 61b) extrémités, destiné à être disposé autour d'une portion d'un corps vertébral (C), un support de fixation (20, 31, 48) ayant des parties avant (23, 33, 50) et arrière (34, 51), **caractérisé en ce qu'**il comprend une zone d'attache (25, 34, 51) à laquelle ladite première extrémité (27a, 47a, 61a) est solidarisée ou apte à être solidarisée, un premier élément de blocage (24, 32, 49), monté sur ledit support de fixation (20, 31, 48) en sorte d'être apte à translater entre lesdites parties avant (23, 33, 50) et arrière (34, 51), délimitant des zones de passage avant (30, 35, 52) et arrière (29, 36, 53) avec lesdites parties avant et arrière, respectivement, permettant le passage de ladite seconde extrémité libre, et un moyen d'appui et de guidage (25, 39, 56) dudit élément longiligne, **en ce qu'**en fonctionnement la seconde extrémité libre passe au moins une fois dans lesdites zones avant et arrière et sur ledit moyen d'appui et de guidage (25, 39, 56) en passant autour, au moins partiellement, d'une tige de liaison (T) et d'une portion d'un corps vertébral (C) pour la solidarisation amovible de ladite tige de liaison (T) avec ledit support de fixation (20, 31, 48), et **en ce que** la traction exercée sur la seconde extrémité libre permet le déplacement du premier élément de blocage (24, 32, 49) vers la partie avant (23, 33,50) et corrélativement le rapprochement de ladite portion du corps vertébral (C) vers ladite tige de liaison (T).

2. Dispositif (46, 60) selon la revendication 1, **caractérisé en ce que** le support de fixation (31, 48) comporte une partie (37, 54) ayant une forme générale semi-cylindrique ouverte formant un logement (38, 55) apte à recevoir une tige de liaison (T).

3. Dispositif (46, 60) selon la revendication 2, **caractérisé en ce que** ladite partie (37, 54) de forme générale semi-cylindrique ouverte comprend une portion (39, 56) de sa surface externe faisant office dudit moyen de guidage et d'appui pour ledit élément longiligne.

4. Dispositif (46, 60) selon l'une quelconque des revendications 1 à 3, **caractérisé en ce que** la partie arrière (34, 51) fait office de ladite zone d'attache.

5. Dispositif (26) selon la revendication 1, **caractérisé en ce qu'**il comprend un second élément de blocage (25) monté sur ledit support (20) en sorte d'être apte à translater entre les parties avant (23) et arrière délimitant avec le premier élément de blocage (24) une zone de passage intermédiaire (28), ledit second élément de blocage (25) faisant office dudit moyen d'appui et de guidage pour l'élément longiligne.

6. Dispositif selon la revendication 5, **caractérisé en ce que** le second élément de blocage (25) fait office de ladite zone d'attache.

7. Dispositif (26, 46, 60) selon l'une quelconque des revendications 1 à 6, **caractérisé en ce que** le support de fixation (20, 31, 48) comprend deux parties latérales (40, 41, 58, 59) entre lesquelles ledit premier élément de blocage (24, 32, 49), éventuellement ledit second élément de blocage (25), est monté coulissant.

8. Dispositif (26, 46, 60) selon la revendication 7, **caractérisé en ce que** la distance interne (d) séparant les parties latérales (40, 41, 58, 59) est de l'ordre de celle de la largeur principale (l) dudit élément longiligne (27, 47, 61) ou inférieure.

9. Dispositif (26) selon l'une quelconque des revendications 1 à 8, **caractérisé en ce que** la partie avant (23) du support de fixation (26) est dimensionnée et agencée par rapport au premier élément de blocage (24) en sorte de faire office de butée avant pour ledit premier élément de blocage, de préférence les bords gauche et droit de la partie avant se projetant de part et d'autre des parties latérales font office de butée aux bords gauche et droit du premier élément de blocage.

10. Dispositif (26, 60) selon l'une quelconque des revendications 7 à 9, **caractérisé en ce que** le premier élément de blocage (24, 49), éventuellement le second élément de blocage (25), comprend une ouverture centrale (21, 22) dont les bords latéraux internes sont ménagés en sorte de recevoir les parties latérales (58, 59) du support de fixation (20, 48) et faire office de guide pour la coulisse du premier élément de blocage (24, 49), éventuellement du second élément de blocage (25), sur les parties latérales (58, 59).

11. Dispositif (26, 60) selon la revendication 10, **caractérisé en ce que** la périphérie supérieure (21a) de l'ouverture centrale (21) débouchant vers la partie avant (23) est délimitée par des bords gauche (21b) et droit (21c) et **en ce que** le bord gauche (21b) se projette de ladite périphérie supérieure (21a) en sorte que seul le bord gauche (21b) du premier élément de blocage (24) vienne en butée contre la partie avant du support de fixation (26,60) ménageant un jeu entre le bord droit (21c) et la partie avant (23).

12. Dispositif (26, 60) selon l'une ou l'autre des revendications 10 et 11, **caractérisé en ce que** le premier élément de blocage (24, 49), éventuellement le second élément de blocage (25), comprend des rainures verticales, orientées de la partie arrière vers la partie avant (23), et débouchant sur la périphérie supérieure (21a), et éventuellement la périphérie inférieure (21b), de l'ouverture centrale (21) en sorte de ménager des dents de pincement sur la périphérie supérieure, et éventuellement la périphérie inférieure.

13. Dispositif selon l'une ou l'autre des revendications 10 et 11, **caractérisé en ce que** l'ouverture centrale du premier élément de blocage, éventuellement du second élément de blocage, débouchant vers la partie avant du support de fixation comporte selon au moins l'un de ses bords gauche ou droit des dents en forme de dents de scie.

14. Dispositif selon l'une quelconque des revendications précédentes, **caractérisé en ce que** le support de fixation est dans un matériau sélectionné seul ou en combinaison parmi les matériaux suivants : acier inoxydable, titane, céramique, polypropylène, polyéthylène, polyéthylène haute densité, le polyétheréthercétone (PEEK), polyéthercétonecétone (PEKK).

15. Dispositif selon l'une quelconque des revendications précédentes, **caractérisé en ce que** l'élément longiligne est obtenu par la mise en forme de fils multi-filamentaires et/ou de monofilaments par tissage, tricotage ou tressage, de préférence par tressage de fils multi-filamentaires.

16. Dispositif selon l'une quelconque des revendications précédentes, **caractérisé en ce que** l'élément longiligne est une tresse tubulaire dont les première et seconde extrémités ont été cousues pour la mise en forme à plat de la tresse tubulaire.

17. Dispositif selon l'une quelconque des revendications précédentes, **caractérisé en ce que** la seconde extrémité est confectionnée en forme de pointe, de préférence comporte une boucle de préhension.

18. Dispositif selon l'une quelconque des revendications précédentes, **caractérisé en ce que** l'élément longiligne comprend une zone centrale disposée entre des zones latérales et **en ce que** les zones latérales présentent un coefficient de friction moins élevé que la zone centrale.

## Patentansprüche

1. Implantierbare Wirbelbefestigungsvorrichtung (26, 46, 60), insbesondere zur Osteosynthese von Wirbeln und zur Behandlung von Skoliosen, umfassend ein im Wesentlichen flaches längliches Element (27, 47, 61) von vorbestimmter Hauptbreite (l), das ein erstes (27a, 47a, 61a) und zweites (27b, 47b, 61b) Ende aufweist, das dazu bestimmt ist, um einen Abschnitt eines Wirbelkörpers (C) herum angeordnet zu werden, und einen Befestigungsträger (20, 31, 48), der einen vorderen (23, 33, 50) und einen hinteren Teil (34, 51) aufweist,
**dadurch gekennzeichnet,**
**dass** sie einen Befestigungsbereich (25, 34, 51), mit dem das erste Ende (27a, 47a, 61a) fest verbunden ist oder fest verbunden werden kann, ein erstes Blockierelement (24, 32, 49), das an dem Befestigungsträger (20, 31, 48) derart befestigt ist, um geeignet zu sein, zwischen dem vorderen (23, 33, 50) und hinteren (34, 51) Teil verschoben zu werden, wodurch ein vorderer (30, 35, 52) und hinter Durchgangsbereich (29, 36, 53) mit dem vorderen und hinteren Teil begrenzt wird, die jeweils den Durchgang des zweiten freien Endes ermöglichen, und ein Stütz- und Führungsmittel (25, 39, 56) des länglichen Elements aufweist,
**dass** im Betrieb das zweite freie Ende mindestens einmal durch den vorderen und hinteren Bereich durchgeht und über das Stütz- und Führungsmittel (25, 39, 56) geht, indem es mindestens teilweise um eine Verbindungsstange (T) und einen Abschnitt eines Wirbelkörpers (C) zur abnehmbaren Verbindung der Verbindungsstange (T) mit dem Befestigungsträger (20, 31, 48) herumgeht, und
**dass** der Zug, der auf das zweite freie Ende ausgeübt wird, die Verschiebung des ersten Blockierelements (24, 32, 49) in Richtung des vorderen Teils (23, 33, 50) und entsprechend das Annähern des Abschnitts des Wirbelkörpers (C) in Richtung der Verbindungsstange (T) ermöglicht.

2. Vorrichtung (46, 60) gemäß Anspruch 1, **dadurch gekennzeichnet, dass** der Befestigungsträger (31, 48) einen Teil (37, 54) aufweist, der eine allgemein halbzylindrische offene Form aufweist, die eine Aufnahme (38, 55) bildet, die geeignet ist, eine Verbindungsstange (T) aufzunehmen.

3. Vorrichtung (46, 60) gemäß Anspruch 2, **dadurch gekennzeichnet, dass** der Teil (37, 54) von allgemein halbzylindrischer offener Form einen Abschnitt (39, 56) seiner Außenfläche aufweist, der als das Führungs- und Stützmittel für das längliche Element dient.

4. Vorrichtung (46, 60) gemäß einem der Ansprüche 1 bis 3, **dadurch gekennzeichnet, dass** der hintere Teil (34, 51) als der Befestigungsbereich dient.

5. Vorrichtung (26) gemäß Anspruch 1, **dadurch gekennzeichnet, dass** sie ein zweites Blockierelement (25) aufweist, das auf dem Träger (20) derart befestigt ist, um geeignet zu sein, zwischen dem vorderen (23) und dem hinteren Teil verschoben zu werden, wodurch es mit dem ersten Blockierelement (24) einen Zwischendurchgangsbereich (28) begrenzt, wobei das zweite Blockierelement (25) als das Stütz- und Führungsmittel für das längliche Element dient.

6. Vorrichtung gemäß Anspruch 5, **dadurch gekennzeichnet, dass** das zweite Blockierelement (25) als der Befestigungsbereich dient.

7. Vorrichtung (26, 46, 60) gemäß einem der Ansprüche 1 bis 6, **dadurch gekennzeichnet, dass** der Befestigungsträger (20, 31, 48) zwei seitliche Teile (40, 41, 58, 59) aufweist, zwischen denen das erste Blockierelement (24, 32, 49), eventuell auch das zweite Blockierelement (25), verschiebbar befestigt ist.

8. Vorrichtung (26, 46, 60) gemäß Anspruch 7, **dadurch gekennzeichnet, dass** der innere Abstand (d), der die seitlichen Teile (40, 41, 58, 59) trennt, in der Größenordnung von jenem der Hauptbreite (l) des länglichen Elements (27, 47, 61) oder niedriger ist.

9. Vorrichtung (26) gemäß einem der Ansprüche 1 bis 8, **dadurch gekennzeichnet, dass** der vordere Teil (23) des Befestigungsträgers (26) in Bezug auf das erste Blockierelement (24) derart dimensioniert und angeordnet ist, um als vorderer Anschlag für das erste Blockierelement zu dienen, wobei der linke und rechte Rand des vorderen Teils, die auf beiden Seiten der seitlichen Teile vorstehen, vorzugsweise als Anschlag an dem linken und rechten Rand des ersten Blockierelements dienen.

10. Vorrichtung (26, 60) gemäß einem der Ansprüche 7 bis 9, **dadurch gekennzeichnet, dass** das erste Blockierelement (24, 49), eventuell das zweite Blockierelement (25), eine mittlere Öffnung (21, 22) aufweist, deren seitliche Ränder derart ausgebildet sind, um die seitlichen Teile (58, 59) des Befestigungsträgers (20, 48) aufzunehmen und als Führung für das Gleiten des ersten Blockierelements (24, 49), eventuell des zweiten Blockierelements (25), auf den seitlichen Teilen (58, 59) zu dienen.

11. Vorrichtung (26, 60) gemäß Anspruch 10, **dadurch gekennzeichnet, dass** der obere Umfang (21a) der mittleren Öffnung (21), die in Richtung des vorderen Teils (23) mündet, durch einen linken (21a) und rechten Rand (21c) begrenzt ist und dadurch, dass der linke Rand (21b) von dem oberen Umfang (21a) derart vorsteht, dass nur der linke Rand (21b) des ersten Blockierelements (24) in Anschlag gegen den vorderen Teil des Befestigungsträgers (26, 60) kommt, wodurch ein Spiel zwischen dem rechten Rand (21c) und dem vorderen Teil (23) bestehen bleibt.

12. Vorrichtung (26, 60) gemäß einem der Ansprüche 10 und 11, **dadurch gekennzeichnet, dass** das erste Blockierelement (24, 49), eventuell auch das zweite Blockierelement (25), vertikale Rillen aufweist, die von dem hinteren Teil zu dem vorderen Teil (23) ausgerichtet sind und auf dem oberen Umfang (21a) und eventuell dem unteren Umfang (21b) der mittleren Öffnung (21) derart münden, um Klemmzähne auf dem oberen Umfang und eventuell dem unteren Umfang bestehen zu lassen.

13. Vorrichtung gemäß einem der Ansprüche 10 und 11, **dadurch gekennzeichnet, dass** die mittlere Öffnung des ersten Blockierelements, eventuell des zweiten Blockierelements, die in Richtung des vorderen Teils des Befestigungsträgers mündet, übereinstimmend mit mindestens einem ihres rechten oder linken Randes Zähne in Form von Sägezähnen aufweist.

14. Vorrichtung gemäß einem der vorhergehenden Ansprüche, **dadurch gekennzeichnet, dass** der Befestigungsträger aus einem Material besteht, das allein oder in Kombination aus den folgenden Materialien ausgewählt ist: Edelstahl, Titan, Keramik, Polypropylen, Polyethylen, Polyethylen hoher Dichte, Polyetheretherketon (PEEK), Polyetherketonketon (PEKK).

15. Vorrichtung gemäß einem der vorhergehenden Ansprüche, **dadurch gekennzeichnet, dass** das längliche Element durch das Formen von Multifilamentgarnen und/oder Monofilamentgarnen durch Weben, Stricken oder Flechten, vorzugsweise durch Flechten von Multifilamentgarnen erhalten ist.

16. Vorrichtung gemäß einem der vorhergehenden Ansprüche, **dadurch gekennzeichnet, dass** das längliche Element ein rohrförmiges Geflecht ist, dessen erstes und zweites Ende zur flachen Formgebung des rohrförmigen Geflechts vernäht worden sind.

17. Vorrichtung gemäß einem der vorhergehenden Ansprüche, **dadurch gekennzeichnet, dass** das zweite Ende in Form von einer Spitze angefertigt ist, vorzugsweise eine Greifschlaufe aufweist.

18. Vorrichtung gemäß einem der vorhergehenden Ansprüche, **dadurch gekennzeichnet, dass** das längliche Element einen mittleren Bereich aufweist, der zwischen seitlichen Bereichen angeordnet ist, und dadurch, dass die seitlichen Bereiche einen niedrigeren Reibungskoeffizienten als der mittlere Bereich aufweisen.

## Claims

1. An implantable spinal setting device (26, 46, 60), particularly for spinal osteosynthesis and the treatment of scolioses, comprising a substantially flat elongated element (27, 47, 61) of predetermined main width (I), having first (27a, 47a, 61a) and second (27b, 47b, 61b) ends, designed to be disposed around a portion of a vertebral body (C), a setting support (20, 31, 48) having front (23, 33, 50) and rear (34, 51) portions, **characterized in that** it comprises an attachment zone (25, 34, 51) to which said first end (27a, 47a, 61a) is firmly attached or capable of being firmly attached, a first blocking element (24, 32, 49), mounted on said setting support (20, 31, 48) so as to be capable of translating between said front (23, 33, 50) and rear (34, 51) portions, delimiting front (30, 35, 52) and rear (29, 36, 53) passage zones with said front and rear portions, respectively, allowing passage of said second free end, and a support and guidance means (25, 39, 56) of said elongated element, **in that** in operation the second free end passes at least once in said front and rear zones and on said support and guidance means (25, 39, 56) by passing around, at least partially, a connecting rod (T) and a portion of a vertebral body (C) for the removable firm attachment of said connecting rod (T) with said setting support (20, 31, 48), and **in that** the tension exerted on the second free end allows the displacement of the first blocking element (24, 32, 49) toward the front portion (23, 33, 50) and, correlatively, the convergence of said portion of the vertebral body (C) toward said connecting rod (T).

2. The device (46, 60) according to claim 1, **characterized in that** the setting support (31, 48) includes a portion (37, 54) having a generally open semi-cylindrical shape forming a recess (38, 55) capable of receiving a connecting rod (T).

3. The device (46, 60) according to claim 2, **characterized in that** said portion (37, 54) with a generally open semi-cylindrical shape comprises a portion (39, 56) of its external surface serving as said means of guidance and support for said elongated element.

4. The device (46, 60) according to any one of claims 1 to 3, **characterized in that** the rear portion (34, 51) serves as said attachment zone.

5. The device (26) according to claim 1, **characterized in that** it comprises a second blocking element (25) mounted on said support (20) so as to be able to translate between the front (23) and rear portions, delimiting with the first blocking element (24) an intermediate passage zone (28), said second blocking element (25) serving as said means of support and guidance for the elongated element.

6. The device according to claim 5, **characterized in that** the second blocking element (25) serves as said attachment zone.

7. The device (26, 46, 60) according to any one of claims 1 to 6, **characterized in that** the setting support (20, 31, 48) comprises two lateral portions (40, 41, 58, 59) between which said first blocking element (24, 32, 49), possibly said second blocking element (25) is mounted sliding.

8. The device (26, 46, 60) according to claim 7, **characterized in that** the internal distance (d) separating the lateral portions (40, 41, 58, 59) is on the order of that of the main width (I) of said elongated element (27, 47, 61) or less.

9. The device (26) according to any one of claims 1 to 8, **characterized in that** the front portion (23) of the setting support (26) is dimensioned and arranged with respect to the first blocking element (24) so as to serve as a front abutment for said first blocking element, the left and right edges of the front portion preferably projecting on either side of the lateral portions serve as abutments to the left and right edges of the first blocking element.

10. The device (26, 60) according to any one of claims 7 to 9, **characterized in that** the first blocking element (24, 49), possibly the second blocking element (25), comprises a central opening (21, 22) of which the lateral internal edges are arranged so as to receive the lateral portions (58, 59) of the setting support (20, 48) and to serve as guides for the sliding of the first blocking element (24, 49), possibly the second blocking element (25), on the lateral portions (58, 59).

11. The device (26, 60) according to claim 10, **characterized in that** the upper periphery (21a) of the central opening (21) leading to the front portion (23) is delimited by the left (21b) and right (21c) edges and **in that** the left edge (21b) projects from said upper periphery (21a) so that only the left edge (21b) of the first blocking element (24) comes into abutment against the front portion of the setting support (26,60), providing clearance between the right edge (21c) and the front portion (23).

12. The device (26, 60) according to one or another of claims 10 and 11, **characterized in that** the first blocking element (24, 49), possibly the second blocking element (25), comprises vertical slots, oriented from the rear portion toward the front portion (23), and leading to the upper periphery (21a), and possibly the lower periphery (21b), from the central opening (21) so as to provide pinching teeth on the upper periphery, and possibly the lower periphery.

13. The device according to one or another of claims 10 and 11, **characterized in that** the central opening of the first blocking element, possibly of the second blocking element, leading toward the front portion of the setting support includes, along at least one of its left or right edges, teeth shaped like saw teeth.

14. The device according to any one of the preceding claims, **characterized in that** the setting support is made of a material selected alone or in combination among the following materials: stainless steel, titanium, ceramic, polypropylene, polyethylene, high density polyethylene, polyether ether ketone (PEEK), polyetherketoneketone (PEKK).

15. The device according to any one of the preceding claims, **characterized in that** the elongated element is obtained by the forming of multifilament and/or monofilament yarns by weaving, knitting or braiding, preferably by braiding multifilament yarns.

16. The device according to any one of the preceding claims, **characterized in that** the elongated element is a tubular braid of which the first and second ends have been sewn for the flat forming of the tubular braid.

17. The device according to any one of the preceding claims, **characterized in that** the second end is manufacture in the form of a point, preferably including a gripping loop.

18. The device according to any one of the preceding claims, **characterized in that** the elongated element comprises a central zone disposed between lateral zones and **in that** the lateral zones have a smaller coefficient of friction than the central zone.
